# EUROPEAN PATENT SPECIFICATION

(11) **EP 2 764 087 B1**
(45) Date of publication and mention of the grant of the patent: **31.05.2017**
(21) Application number: 12780430.0
(22) Date of filing: 07.10.2012
(51) Int. Cl.: C12N 1/20, C12P 7/06, C12P 7/54, C12P 7/56, C12R 1/01

(54) **VERSATILE EXTREMELY THERMOPHILIC BACTERIA FOR THE CONVERSION OF BIOMASS**
VIELSEITIGE EXTREM THERMOPHILE BAKTERIEN ZUR UMWANDLUNG VON BIOMASSE
BACTÉRIES VERSATILES EXTRÊMEMENT THERMOPHILES POUR LA CONVERSION DE BIOMASSE

(30) Priority: 07.10.2011 US 201161544831 P; 07.10.2011 EP 11008155; 07.11.2011 US 201161556448 P; 07.11.2011 EP 11008857; 10.07.2012 EP 12175684; 10.07.2012 US 201261669998 P
(43) Date of publication of application: 13.08.2014
(73) Proprietor: Direvo Industrial Biotechnology GmbH, 50829 Köln (DE)
(72) Inventor: SVETLITCHNYI, Vitaly, 50829 Köln (DE); CURVERS, Simon, 50829 Köln (DE)
(74) Representative: Roth, Andy Stefan
(86) International application number: PCT/EP2012/069808
(87) International publication number: WO 2013/050582

(56) References cited:
- WO-A1-2007/134607
- LARSEN L ET AL: "Thermoanaerobacter mathranii sp. nov., an ethanol-producing, extremely thermophilic anaerobic bacterium from a hot spring in Iceland", ARCHIVES OF MICROBIOLOGY, SPRINGER, DE, vol. 168, no. 2, 1 January 1997 (1997-01-01), pages 114-119, XP002238104, ISSN: 0302-8933, DOI: 10.1007/S002030050476 cited in the application -& DATABASE EMBL [Online] 20 August 1997 (1997-08-20), "T.mathranii 16S rRNA gene", XP002690368, retrieved from EBI accession no. EM_STD:Y11279 Database accession no. Y11279
- RAINEY F A ET AL: "PHYLOGENETIC ANALYSIS OF ANAEROBIC THERMOPHILIC BACTERIA: AID FOR THEIR RECLASSIFICATION", JOURNAL OF BACTERIOLOGY, AMERICAN SOCIETY FOR MICROBIOLOGY, WASHINGTON, DC; US, vol. 175, no. 15, 1 August 1993 (1993-08-01), pages 4772-4779, XP000564368, ISSN: 0021-9193 -& DATABASE EMBL [Online] 7 January 1994 (1994-01-07), "Thermoanaerobacter thermohydrosulfuricus (DSM 567) 16S ribosomal RNA (16S rRNA) gene.", XP002690370, retrieved from EBI accession no. EM_STD:L09161 Database accession no. L09161
- YONG-EOK LEE ET AL: "Taxonomic Distinction of Saccharolytic Thermophilic Anaerobes: Description of Thermoanaerobacterium xylanolyticum gen. nov. , sp. ~ nov., and Thermoanaerobacterium saccharolyticum gen. nov., sp. nov. ; Reclassification of Thermoanaerobium brockii, Clostridium thermosulfurogenes, and Clostridium ther", INT J SYST BACTERIOL, vol. 43, no. 1, 1 January 1993 (1993-01-01), pages 41-51, XP055049646, cited in the application
- BALAT M ED - BALAT M: "Production of bioethanol from lignocellulosic materials via the biochemical pathway: A review", ENERGY CONVERSION AND MANAGEMENT,, vol. 52, 6 September 2010 (2010-09-06), pages 858-875, XP027505067,

### SEQUENCE LISTING

<110> DIREVO Industrisl Biotechnology GmbH
<120> VERSATILE EXTREMELY THERMOPHILIC BACTERIA FOR CONVERSION OF BIOMASS
<130> DIR-PA15-PCT
<150> US61/669,998
   <151> 2012-07-10
   <150> EP12175684
   <151> 2012-07-10
   <150> EP11008857
   <151> 2011-11-07
   <150> US61/556,448
   <151> 2011-11-07
   <150> US61/544,831
   <151> 2011-10-07
   <150> EP11008155
   <151> 2011-10-07
<160> 10
<170> BiSSAP 1.2
<210> 1
   <211> 1422
   <212> DNA
   <213> Thermoanaerobacter sp.
<220>
   <221> source
   <222> 1..1422
   <223> /organism="Thermoanaerobacter sp." /note="16SrDNA consensus sequence for Thermoanaerobacter sp. DIB004G " /mol_type="unassigned DNA"
<400> 1
   ggttgggtca ccggcttcgg gtgtcgcagg ctctcgtggt gtgacgggcg gtgtgtacaa      60
   ggcccgggaa cgtattcacc gcggcatgct gatccgcgat tactagcgat tccgacttca     120
   tgcaggcgag ttgcagcctg caatccgaac ttggaccggc tttttgggat tcgctccgcc     180
   tcacggcttc gcttccctct gtaccggcca ttgtagcacg tgtgtggccc agggcattta     240
   gggcatgatg atttgacgtc atccccacct tcctccgtgt cctccacggc agtccctcta     300
   gagtgcccgg cttacccgct ggcaactaga ggcaggggtt gcgctcgttg cgggacttaa     360
   cccaacatct cacgacacga gctgacgaca accatgcacc acctgtgcag gctccttacc     420
   tcccggtaag gtcgctcccc tttcggttcg ctactacctg catgtcaagc cctggtaagg     480
   ttcttcgcgt tgcttcgaat taaaccacat gctccaccgc ttgtgcgggc ccccgtcaat     540
   tcctttgagt ttcaaccttg cggccgtact ccccaggcgg ggtacttatt gcgttcgcta     600
   cggcacggaa cgcttccgcg ccccacacct agtacccatc gtttacagcg tggactacca     660
   gggtatctaa tcctgttcgc tccccacgct ttcgcgcctc agcgtcaggg ccagtccaga     720
   gagtcgcctt cgccactggt attcctcccg atatctacgc atttcaccgc tacaccggga     780
   attccactcc cctctcctgc cctctagcca atcagtttca gatgctaccc cccggttgag     840
   cccgggtctt ttacacctga cttgattgac cgcctacgcg ccctttacgc ccagtaattc     900
   cggacaacgc tcgcccccta cgtcttaccg cggctgctgg cacgtagtta gccggggctt     960
   tcgtgtggta ccgtcatccc ttcttcccac actaacgggg tttacaaccc gaaggccttc    1020
   ctcccccacg cggcgtcgct gggtcaggct tccgcccatt gcccaagatt ccccactgct    1080
   gcctcccgta ggagtctggg ccgtgtctca gtcccagtgt ggccgtccac cctctcaggc    1140
   cggctacccg tcgtcgcctt ggtaggccgt taccctacca actagctgat gggacgcggg    1200
   cccatcctta agcggtagct tgcgcttccc tttcctccct ataggatgcc ctataaggag    1260
   cttatccagt attaccaccc ctttcgaggt gctatcccgg tcttaagggt aggttgccca    1320
   cgcgttactc acccgtccgc cgctatccgc cacccaacta cgttgagtgc cggaccgctc    1380
   gactgcatgt gttaggcacg ccgccagcgt tcgtcctgag cc                       1422
<210> 2
   <211> 1659
   <212> DNA
   <213> Thermoanaerobacter sp.
<220>
   <221> source
   <222> 1..1659
   <223> /organism="Thermoanaerobacter sp." /note="16SrDNA consensus sequence for Thermoanaerobacter sp. DIB087G " /mol_type="unassigned DNA"
<400> 2
   actcaagtgg gcacgttttt ttctcttcat cacgtttcta acatgcccac ttgagtgccg      60
   ggttgggtca ccggcttcgg gtgttgcaga ctctcgtggt gtgacgggcg gtgtgtacaa     120
   ggcccgggaa cgtattcacc gcggcatgct gatccgcgat tactagcgat tccgacttca     180
   tgcaggcgag ttgcagcctg caatccgaac ttggaccggc tttttggggt ccgctccaga     240
   tcgctccttc gcctccctct gtaccggcca ttgtagcacg tgtgtggccc agggcatata     300
   gggcatgatg atttgacgtc atccccacct tcctccgtgt tgtccacggc agtccctcta     360
   gagtgcctcc gtcactcaac tgaacacgct atcccttcct ctctactctt tcctaacatg     420
   ttcagttgag tgacggactg gcaactagaa gcaagggttg cgctcgttgc gggacttaac     480
   ccaacatctc acgacacgag ctgacgacaa ccatgcacca cctgtgcagg ctcccggcac     540
   tcaagtaggc acttcattct ccctcttact accttctcta tcatgcccac ttgagtgccg     600
   ggtcgctcac ctttcggctc gctactacct gcatgtcaag ccctggtaag gttcttcgcg     660
   ttgcttcgaa ttaaaccaca tgctccaccg cttgtgcggg cccccgtcaa ttcctttgag     720
   tttcaacctt gcggccgtac tccccaggcg gggtacttat tgcgttaact acggcacgga     780
   atgcttccgc atcccacacc tagtacccat cgtttacggc gtggactacc agggtatcta     840
   atcctgtttg ctccccacgc tttcgcgcct cagcgtcagg gtcagtccag agagtcgcct     900
   tcgccactgg tattcctccc gatatctacg catttcaccg ctacaccggg aattccactc     960
   ccctctcctg ccctctagcc acccagtttc atgtgcatcc cccgggttga gcccgggttt    1020
   tttacacctg acttaagtgg ccgcctacgc gccctttacg cccagtaatt ccggacaacg    1080
   ctcgccccct acgtcttacc gcggctgctg gcacgtagtt agccggggct ttcgtgtggt    1140
   accgtcatct attcttccca cactatcgag ctttacgacc cgaaggcctt cttcgctcac    1200
   gcggcgtcgc tgcgtcaggc tttcgcccat tgcgcaagat tccccactgc tgcctcccgt    1260
   aggagtctgg gccgtgtctc agtcccagtg tggccgacca ccctctcagg ccggctaccc    1320
   gtcgtcgcct tggtaggccg ttaccctacc aactagctga tgggacgcgg gcccatcctt    1380
   aagcggtagc ttccgctacc ttccctcctc ataggatgcc ctacaaggag cttatccagt    1440
   attagcaccc ctttcgaggt gttatcccgg tcttaagggt aggttgccca cgcgttactc    1500
   acccgtccgc cgctatccgg cactcaactc cgtgcttacc ttactttgca ccacttttat    1560
   tactttcttc ttctactata cttccttccc cttaagtaag cacttagttg agtgccggac    1620
   cgctcgactt gcatgtgtta ggcacgccgc cagcgttcg                           1659
<210> 3
   <211> 1436
   <212> DNA
   <213> Thermoanaerobacter sp.
<220>
   <221> source
   <222> 1..1436
   <223> /organism="Thermoanaerobacter sp." /note="16SrDNA consensus sequence for Thermoanaerobacter sp. DIB097X " /mol_type="unassigned DNA"
<400> 3
   cccggttggg tcaccggctt cgggtgtcgc aggctctcgt ggtgtgacgg gcggtgtgta 60
   caaggcccgg gaacgtattc accgcggcat gctgatccgc gattactagc gattccgact 120
   tcatgcaggc gagttgcagc ctgcaatccg aacttggacc ggctttttgg gattcgctcc 180
   gcctcgcggc ttcgctcccc tctgtaccgg ccattgtagc acgtgtgtgg cccagggcat 240
   atagggcatg atgatttgac gtcatcccca ccttcctccg tgtcctccac ggcagtcccc 300
   ctagagtgcc cggcttaccc gctggcaact agaggcaggg gttgcgctcg ttgcgggact 360
   taacccaaca tctcacgaca cgagctgacg acaaccatgc accacctgtg caggctcctt 420
   acctcccggt aaggtcgctc ccctttcggt tcgctactac ctgcatgtca agccctggta 480
   aggttcttcg cgttgcttcg aattaaacca catgctccac cgcttgtgcg ggcccccgtc 540
   aattcctttg agtttcaacc ttgcggccgt actccccagg cggggtactt attgcgttcg 600
   ctacggcacg gaacgcttcc gcgccccaca cctagtaccc atcgtttaca gcgtggacta 660
   ccagggtatc taatcctgtt cgctccccac gctttcgcgc ctcagcgtca gggccagtcc 720
   agagagtcgc cttcgccact ggtattcctc ccgatatcta cgcatttcac cgctacaccg 780
   ggaattccac tcccctctcc tgccctctag ccaatcagtt tcagatgcta cccccgggtt 840
   gagcccgggt cttttacacc tgacttgatt gaccgcctac gcgcccttta cgcccagtaa 900
   ttccggacaa cgctcgcccc ctacgtctta ccgcggctgc tggcacgtag ttagccgggg 960
   ctttcgtgtg gtaccgtcat cccttcttcc cacactaacg gggtttacaa cccgaaggcc 1020
   ttcctccccc acgcggcgtc gctgggtcag gcttccgccc attgcccaag attccccact 1080
   gctgcctccc gtaggagtct gggccgtgtc tcagtcccag tgtggccgac caccctctca 1140
   ggccggctac ccgtcgtcgc cttggtaggc cgttacccta ccaactagct gatgggacgc 1200
   gggcccatcc ttaagcggta gcttgcgcct ccctttcctc cctataggat gccctataag 1260
   gagcttatcc agtattacca cccctttcga ggtgctatcc cggtcttaag ggtaggttgc 1320
   ccacgcgtta ctcacccgtc cgccgctatc cgccacccaa ctacgttgag tgccggaccg 1380
   ctcgacttgc atgtgttagg cacgccgcca gcgttcgtcc tgagccatga tcaaac 1436
<210> 4
   <211> 1080
   <212> DNA
   <213> Thermoanaerobacter sp.
<220>
   <221> source
   <222> 1..1080
   <223> /organism="Thermoanaerobacter sp." /note="16SrDNA consensus sequence for Thermoanaerobacter sp. DIB101G " /mol_type="unassigned DNA"
<400> 4
   gctcaggacg aacgctggcg gcgtgcctaa cacatgcaag tcgagcggtc cggcactcaa      60
   ctaagtgctt acttaagggg aaggaagtat agtagaagaa gaaggtaata aaagtgatgc     120
   aaagtaaggt aagcacggag ttgagtgccg gatagcggcg gacgggtgag taacgcgtgg     180
   gcaacctacc cttaagaccg ggataacacc tcgaaagggg tgctaatact ggataagctc     240
   cttgtagggc atcctatgag gagggaaggt agcggaagct accgcttaag gatgggcccg     300
   cgtcccatca gctagttggt agggtaacgg cctaccaagg cgacgacggg tagccggcct     360
   gagagggtgg tcggccacac tgggactgag acacggccca gactcctacg ggaggcagca     420
   gtggggaatc ttgcgcaatg ggcgaaagcc tgacgcagcg acgccgcgtg agcgaagaag     480
   gccttcgggt cgtaaagctc gatagtgtgg gaagaataga tgacggtacc acacgaaagc     540
   cccggctaac tacgtgccag cagccgcggt aagacgtagg gggcgagcgt tgtccggaat     600
   tactgggcgt aaagggcgcg taggcggcca cttaagtcag gtgtaaaaaa cccgggctca     660
   acccggggga tgcacatgaa actgggtggc tagagggcag gagaggggag tggaattccc     720
   ggtgtagcgg tgaaatgcgt agatatcggg aggaatacca gtggcgaagg cgactctctg     780
   gactgaccct gacgctgagg cgcgaaagcg tggggagcaa acaggattag ataccctggt     840
   agtccacgcc gtaaacgatg ggtactaggt gtgggatgcg gaagcattcc gtgccgtagt     900
   taacgcaata agtaccccgc ctggggagta cggccgcaag gttgaaactc aaaggaattg     960
   acgggggccc gcacaagcgg tggagcatgt ggtttaattc gaagcaacgc gaagaacctt    1020
   accagggctt gacatgcagg tagtagcgag ccgaaaggtg agcgacccgg cactcaagtg    1080
<210> 5
   <211> 1451
   <212> DNA
   <213> Thermoanaerobacter sp.
<220>
   <221> source
   <222> 1..1451
   <223> /organism="Thermoanaerobacter sp." /note="16SrDNA consensus sequence for Thermoanaerobacter sp. DIB101X " /mol_type="unassigned DNA"
<400> 5
   gccccacttt cgacggctcc ctccttcccg gttgggtcac cggcttcggg tgtcgcaggc      60
   tctcgtggtg tgacgggcgg tgtgtacaag gcccgggaac gtattcaccg cggcatgctg     120
   atccgcgatt actagcgatt ccgacttcat gcaggcgagt tgcagcctgc aatccgaact     180
   tggaccggct ttttgggatt cgctccgcct cgcggcttcg cttccctctg taccggccat     240
   tgtagcacgt gtgtggccca gggcatatag ggcatgatga tttgacgtca tccccacctt     300
   cctccgtgtc ctccacggca gtccctctag agtgcccggc ttacccgctg gcaactagag     360
   gcaggggttg cgctcgttgc gggacttaac ccaacatctc acgacacgag ctgacgacaa     420
   ccatgcacca cctgtgcagg ctccttacct cccggtaagg tcgctcccct ttcggttcgc     480
   tactacctgc atgtcaagcc ctggtaaggt tcttcgcgtt gcttcgaatt aaaccacatg     540
   ctccaccgct tgtgcgggcc cccgtcaatt cctttgagtt tcaaccttgc ggccgtactc     600
   cccaggcggg gtacttattg cgttcgctac ggcacggaac gcttccgcgc cccacaccta     660
   gtacccatcg tttacagcgt ggactaccag ggtatctaat cctgttcgct ccccacgctt     720
   tcgcgcctca gcgtcagggc cagtccagag agtcgccttc gccactggta ttcctcccga     780
   tatctacgca tttcaccgct acaccgggaa ttccactccc ctctcctgcc ctctagccaa     840
   tcagtttcag atgctacccc cgggttgagc ccgggtcttt tacacctgac ttgattgacc     900
   gcctacgcgc cctttacgcc cagtaattcc ggacaacgct cgccccctac gtcttaccgc     960
   ggctgctggc acgtagttag ccggggcttt cgtgtggtac cgtcatccct tcttcccaca    1020
   ctaacggggt ttacaacccg aaggccttcc tcccccacgc ggcgtcgctg ggtcaggctt    1080
   ccgcccattg cccaagattc cccactgctg cctcccgtag gagtctgggc cgtgtctcag    1140
   tcccagtgtg gccgaccacc ctctcaggcc ggctacccgt cgtcgccttg gtaggccgtt    1200
   accctaccaa ctagctgatg ggacgcgggc ccatccttaa gcggtagctt gcgcctccct    1260
   ttcctcccta taggatgccc tataaggagc ttatccagta ttaccacccc tttcgaggtg    1320
   ctatcccggt cttaagggta ggttgcccac gcgttactca cccgtccgcc gctatccgcc    1380
   acccaactac gttgagtgcc ggaccgctcg acttgcatgt gttaggcacg ccgccagcgt    1440
   tcgtcctgag c                                                         1451
<210> 6
   <211> 1465
   <212> DNA
   <213> Thermoanaerobacter sp.
<220>
   <221> source
   <222> 1..1465
   <223> /organism="Thermoanaerobacter sp." /note="16SrDNA consensus sequence for Thermoanaerobacter sp. DIB103X " /mol_type="unassigned DNA"
<400> 6
   ttcaccccaa tcacctgccc caccttcgac ggctccctcc tccccggttg ggtcaccggc      60
   ttcgggtgtc gcaggctctc gtggtgtgac gggcggtgtg tacaaggccc gggaacgtat     120
   tcaccgcggc atgctgatcc gcgattacta gcgattccga cttcatgcag gcgagttgca     180
   gcctgcaatc cgaacttgga ccggcttttt gggattcgct ccgcctcgcg gcttcgctcc     240
   cctctgtacc ggccattgta gcacgtgtgt ggcccagggc atatagggca tgatgatttg     300
   acgtcatccc caccttcctc cgtgtcctcc acggcagtcc ccctagagtg cccggcttac     360
   ccgctggcaa ctagaggcag gggttgcgct cgttgcggga cttaacccaa catctcacga     420
   cacgagctga cgacaaccat gcaccacctg tgcaggctcc ttacctcccg gtaaggtcgc     480
   tcccctttcg gttcgctact acctgcatgt caagccctgg taaggttctt cgcgttgctt     540
   cgaattaaac cacatgctcc accgcttgtg cgggcccccg tcaattcctt tgagtttcaa     600
   ccttgcggcc gtactcccca ggcggggtac ttattgcgtt cgctacggca cggaacgctt     660
   ccgcgcccca cacctagtac ccatcgttta cagcgtggac taccagggta tctaatcctg     720
   ttcgctcccc acgctttcgc gcctcagcgt cagggccagt ccagagagtc gccttcgcca     780
   ctggtattcc tcccgatatc tacgcatttc accgctacac cgggaattcc actcccctct     840
   cctgccctct agccaatcag tttcagatgc tacccccggg ttgagcccgg gtcttttaca     900
   cctgacttga ttgaccgcct acgcgccctt tacgcccagt aattccggac aacgctcgcc     960
   ccctacgtct taccgcggct gctggcacgt agttagccgg ggctttcgtg tggtaccgtc    1020
   atcccttctt cccacactaa cggggtttac aacccgaagg ccttcctccc ccacgcggcg    1080
   tcgctgggtc aggcttccgc ccattgccca agattcccca ctgctgcctc ccgtaggagt    1140
   ctgggccgtg tctcagtccc agtgtggccg accaccctct caggccggct acccgtcgtc    1200
   gccttggtag gccgttaccc taccaactag ctgatgggac gcgggcccat ccttaagcgg    1260
   tagcttgcgc ctccctttcc tccctatagg atgccctata aggagcttat ccagtattac    1320
   cacccctttc gaggtgctat cccggtctta agggtaggtt gcccacgcgt tactcacccg    1380
   tccgccgcta tccgccaccc aactacgttg agtgccggac cgctcgactt gcatgtgtta    1440
   ggcacgccgc cagcgttcgt cctga                                          1465
<210> 7
   <211> 1665
   <212> DNA
   <213> Thermoanaerobacter sp.
<220>
   <221> source
   <222> 1..1665
   <223> /organism="Thermoanaerobacter sp." /note="16SrDNA consensus sequence for Thermoanaerobacter sp. DIB104X " /mol_type="unassigned DNA"
<400> 7
   actcaagtgg gcacgttttt ttctcttcat cacgtttcta acatgcccac ttgagtgccg      60
   ggttgggtca ccggcttcgg gtgttgcaga ctctcgtggt gtgacgggcg gtgtgtacaa     120
   ggcccgggaa cgtattcacc gcggcatgct gatccgcgat tactagcgat tccgacttca     180
   tgcaggcgag ttgcagcctg caatccgaac ttggaccggc tttttggggt ccgctccaga     240
   tcgctccttc gcctccctct gtaccggcca ttgtagcacg tgtgtggccc agggcatata     300
   gggcatgatg atttgacgtc atccccacct tcctccgtgt tgtccacggc agtccctcta     360
   gagtgcctcc gtcactcaac tgaacacgct atcccttcct ctctactctt tcctaacatg     420
   ttcagttgag tgacggactg gcaactagaa gcaagggttg cgctcgttgc gggacttaac     480
   ccaacatctc acgacacgag ctgacgacaa ccatgcacca cctgtgcagg ctcccggcac     540
   tcaagtaggc acttcattct ccctcttact accttctcta tcatgcccac ttgagtgccg     600
   ggtcgctcac ctttcggctc gctactacct gcatgtcaag ccctggtaag gttcttcgcg     660
   ttgcttcgaa ttaaaccaca tgctccaccg cttgtgcggg cccccgtcaa ttcctttgag     720
   tttcaacctt gcggccgtac tccccaggcg gggtacttat tgcgttaact acggcacgga     780
   atgcttccgc atcccacacc tagtacccat cgtttacggc gtggactacc agggtatcta     840
   atcctgtttg ctccccacgc tttcgcgcct cagcgtcagg gtcagtccag agagtcgcct     900
   tcgccactgg tattcctccc gatatctacg catttcaccg ctacaccggg aattccactc     960
   ccctctcctg ccctctagcc acccagtttc atgtgcatcc cccgggttga gcccgggttt    1020
   tttacacctg acttaagtgg ccgcctacgc gccctttacg cccagtaatt ccggacaacg    1080
   ctcgccccct acgtcttacc gcggctgctg gcacgtagtt agccggggct ttcgtgtggt    1140
   accgtcatct attcttccca cactatcgag ctttacgacc cgaaggcctt cttcgctcac    1200
   gcggcgtcgc tgcgtcaggc tttcgcccat tgcgcaagat tccccactgc tgcctcccgt    1260
   aggagtctgg gccgtgtctc agtcccagtg tggccgacca ccctctcagg ccggctaccc    1320
   gtcgtcgcct tggtaggccg ttaccctacc aactagctga tgggacgcgg gcccatcctt    1380
   aagcggtagc ttccgctacc ttccctcctc ataggatgcc ctacaaggag cttatccagt    1440
   attagcaccc ctttcgaggt gttatcccgg tcttaagggt aggttgccca cgcgttactc    1500
   acccgtccgc cgctatccgg cactcaactc cgtgcttacc ttactttgca ccacttttat    1560
   tactttcttc ttctactata cttccttccc cttaagtaag cacttagttg agtgccggac    1620
   cgctcgactt gcatgtgtta ggcacgccgc cagcgttcgt cctga                    1665
<210> 8
   <211> 1105
   <212> DNA
   <213> Thermoanaerobacter sp.
<220>
   <221> source
   <222> 1..1105
   <223> /organism="Thermoanaerobacter sp." /note="16SrDNA consensus sequence for Thermoanaerobacter sp. DIB107X " /mol_type="unassigned DNA"
<400> 8
   tcaggacgaa cgctggcggc gtgcctaaca catgcaagtc gagcggtccg gcactcaacg      60
   tagttgagtg gcggatagcg gcggacgggt gagtaacgcg tgggcaacct acccttaaga     120
   ccgggatagc acctcgaaag gggtggtaat actggataag ctccttatag ggcatcctat      180
   agggaggaaa gggaagcgca agctaccgct taaggatggg cccgcgtccc atcagctagt     240
   tggtagggta acggcctacc aaggckacga cgggtagccg gcctgagagg gtggtcggcc     300
   acactgggac tgagacacgg cccagactcc tacgggaggc agcagtgggg aatcttgggc     360
   aatgggcgga agcctgaccc agcgacgccg cgtgggggag gaaggccttc gggttgtaaa     420
   ccccgttagt gtgggaagaa gggatgacgg taccacacga aagccccggc taactacgtg     480
   ccagcagccg cggtaagacg tagggggcga gcgttgtccg gaattactgg gcgtaaaggg      540
   cgcgtaggcg gtcaatcaag tcaggtgtaa aagacccggg ctcaacccgg gggtagcacc     600
   tgaaactggt tggctagagg gcaggagagg ggagtggaat tcccggtgta gcggtgaaat     660
   gcgtagatat cgggaggaat accagtggcg aaggcgactc tctggactgg ccctgacgct     720
   gaggcgcgaa agcgtgggga gcgaacagga ttagataccc tggtagtcca cgctgtaaac     780
   gatgggtact aggtgtgggg cgcggaagcg ttccgtgccg tagcgaacgc aataagtacc     840
   ccgcctgggg agtacggccg caaggttgaa actcaaagga attgacgggg gcccgcacaa     900
   gcggtggagc atgtggttta attcgaagca acgcgaagaa ccttaccagg gcttgacatg     960
   caggtggtag cgaaccgaaa ggtgagcgac cttaccggga ggtaaggagc ctgcacaggt    1020
   ggtgcatggt tgtcgtcagc tcgtgtcgtg agatgttggg ttaagtcccg caacgagcgc    1080
   aacccctgcc tctagttgcc agcgg                                          1105
<210> 9
   <211> 20
   <212> DNA
   <213> Artificial Sequence
<220>
   <221> source
   <222> 1..20
   <223> /organism="Artificial Sequence" /note="27F forward Primer" /mol_type="unassigned DNA"
<400> 9
   agagtttgat cmtggctcag 20
<210> 10
   <211> 19
   <212> DNA
   <213> Artificial Sequence
<220>
   <221> source
   <222> 1..19
   <223> /organism="Artificial Sequence" /note="1492R Reverse Primer" /mol_type="unassigned DNA"
<400> 10
   ggttaccttg ttacgactt 19

## Description

### FIELD OF THE DISCLOSURE

The present disclosure pertains to novel isolated xylanolytic, amylolytic and saccharolytic thermophilic bacterial cells belonging to the genus *Thermoanaerobacter,* isolated strains, microbial cultures and compositions comprising said cells.

### BACKGROUND

In general, fermentation products are produced by degradation of starch- containing material into fermentable sugars by liquefaction and saccharification followed by conversion of the sugars directly or indirectly into the desired fermentation product using a fermenting organism.

However, the industry of producing fermentation products such as ethanol and lactic acid is facing the challenge of redirecting the production process from fermentation of relatively easily convertible but expensive starchy materials, to the complex but inexpensive lignocellulosic biomass such as plant biomass.

Unlike starch, which contains homogenous and easily hydrolyzed polymers, lignocellulosic biomass contains variable amounts of cellulose, hemicellulose, lignin and small amounts of protein, pectin, wax and other organic compounds. Lignocellulosic biomass should be understood in its broadest sense, so that it apart from wood, agricultural residues, energy crops also comprises different types of waste from both industry and households. Cellulosic biomass is a vast poorly exploited resource, and in some cases a waste problem. However, hexoses from cellulose can be converted by yeast to fuel ethanol for which there is a growing demand. Pentoses from hemicellulose cannot yet be converted to ethanol commercially but several promising ethanologenic microorganisms with the capacity to convert pentoses and hexoses are under development.

Typically, the first step in utilization of lignocellulosic biomass is a pretreatment step, in order to fractionate the components of lignocellulosic material and increase their surface area. The pretreatment method most often used is steam pretreatment, a process comprising heating of the lignocellulosic material by steam injection to a temperature of 130 - 230°C. Prior to or during steam pretreatment, a catalyst like mineral or organic acid or a caustic agent facilitating disintegration of the biomass structure can be added optionally.

Another type of lignocellulose hydrolysis is acid hydrolysis, where the lignocellulosic material is subjected to an acid such as sulphuric acid whereby the sugar polymers cellulose and hemicellulose are partly or completely hydrolysed to their constituent sugar monomers and the structure of the biomass is destroyed facilitating access of hydrolytic enzymes in subsequent processing steps.

A further method is wet oxidation wherein the material is treated with oxygen at 150-185 °C. Either pretreatment can be followed by enzymatic hydrolysis to complete the release of sugar monomers. This pre-treatment step results in the hydrolysis of cellulose into glucose while hemicellulose is transformed into the pentoses xylose and arabinose and the hexoses glucose, mannose and galactose. Thus, in contrast to starch, the hydrolysis of lignocellulosic biomass results in the release of pentose sugars in addition to hexose sugars. This implies that useful fermenting organisms need to be able to convert both hexose and pentose sugars to desired fermentation products such as ethanol.

After the pre-treatment the lignocellulosic biomass processing schemes involving enzymatic or microbial hydrolysis commonly involve four biologically mediated transformations: (1) the production of saccharolytic enzymes (cellulases and hemicellulases); (2) the hydrolysis of carbohydrate components present in pretreated biomass to sugars; (3) the fermentation of hexose sugars (e.g. glucose, mannose, and galactose); and (4) the fermentation of pentose sugars (e.g., xylose and arabinose).

Each processing step can make the overall process more costly and, therefore, decrease the economic feasibility of producing biofuel or carbon-based chemicals from cellulosic biological material. Thus, there is a need to develop methods that reduce the number of processing steps needed to convert cellulosic biological material to biofuel and other commercially desirable materials.

The four biologically mediated transformations may occur in a single step in a process configuration called consolidated bioprocessing (CBP), which is distinguished from other less highly integrated configurations in that CBP does not involve a dedicated process step for cellulase and/or hemicellulase production. CBP offers the potential for higher efficiency than a processes requiring dedicated cellulase production in a distinct unit operation.

Therefore, the availability of novel microorganisms and methods for converting lignocellulosic biomass material to carbon-based chemicals would be highly advantageous.

### SUMMARY OF THE DISCLOSURE

The present disclosure relates to methods, microorganisms and compositions useful for processing lignocellulosic hydrolysates. The present invention is defined according to the claims and pertains to an isolated *Thermoanaerobacter* sp. cell capable of growing and producing a carbon-based chemical at a temperature of above 70°C comprising a 16S rDNA sequence of SEQ ID NO 1, and wherein the cell is DIB004G deposited under the DSMZ Accession number 25179.

In a first aspect, embodiments of the disclosure provide novel isolated saccharolytic and amylolytic or saccharolytic, amylolytic and xylanolytic, respectively, thermophilic bacterial cells belonging to the genus *Thermoanaerobacter,* in particular capable of producing high levels of ethanol and/or lactic acid from lignocellulosic hydrolysates while producing low levels of acetic acid.

Embodiments of this disclosure relate to an isolated *Thermoanaerobacter* sp. cells comprising a 16S rDNA with a sequence selected form the group consisting of SEQ ID NO. 1, SEQ ID NO. 2, SEQ ID NO. 3, SEQ ID NO. 4, SEQ ID NO. 5, SEQ ID NO. 6, SEQ ID NO. 7 and SEQ ID NO. 8, or homologues thereof.

In one aspect, embodiments of this disclosure relate to the isolated cells of *Thermoanaerobacter* sp. DIB004G, *Thermoanaerobacter* sp. DIB087G, *Thermoanaerobacter* sp. DIB097X, *Thermoanaerobacter* sp. DIB101G, *Thermoanaerobacter* sp. DIB101X, *Thermoanaerobacter* sp DIB103X, *Thermoanaerobacter* sp. DIB DIB104X or *Thermoanaerobacter* sp. DIB107X, each respectively characterized by having a 16S rDNA sequence at least 99 to 100%, preferably 99,5 to 99,99 percent identical to SEQ ID NO. 1, SEQ ID NO. 2, SEQ ID NO. 3, SEQ ID NO. 4, SEQ ID NO. 5, SEQ ID NO. 6, SEQ ID NO. 7 or SEQ ID NO 8 as outlined in table 1.

In still another aspect the present invention relates to an isolated strain comprising a *Thermoanaerobacter* sp.cell according to any of the preceding aspects.

Accordingly, the present disclosure pertains to isolated *Thermoanaerobacter* sp. strains selected from the group consisting of DIB004G, DIB087G, DIB097X, DIB101G, DIB101X, DIB103X, DIB104X and DIB107X, all listed with their respective accession numbers and deposition dates in table 1, cells derived there from, mutants there from, a progenies or homologues.

In one aspect, the disclosure is based on the isolation of the *Thermoanaerobacter* strains DIB097X, DIB101X, DIB103X, DIB104X and DIB107X, which are capable of growing and producing high levels of carbon based fermentation products from lignocellulosic hydrolysates and/or directly from poly-, oligo, di- and/or monosaccharides, in particular from poly-, oligo, di- and/or monosaccharides derived from pre-treated lignocellulosic biomass with polysaccharides being limited to hemicelluloses, e.g. xylan and starch.

The disclosure is further based on the isolation of the *Thermoanaerobacter* sp. strains DIB004G, DIB087G and DIB101G which are capable of growing and producing high levels of carbon based fermentation products from lignocellulosic hydrolysates and/or directly from poly- , oligo, di- and/or monosaccharides, in particular from poly-, oligo, di- and/or monosaccharides derived from pre-treated lignocellulosic biomass with polysaccharides being limited to starch.

One of the advantages of the microorganisms according to the present disclosure and mutants thereof are the broad substrate specificity, since they are capable of utilizing pentoses such as xylose and arabinose and of hexoses such as glucose, mannose, fructose and galactose. The strains further have the advantage of being extremely thermophilic and thus are capable of growing at very high temperatures resulting in high productivities and substrate conversion rates, low risk of contamination and facilitated product recovery.

In still another aspect, embodiments of this disclosure relate to an isolated *Thermoanaerobacter* sp. cell comprising a 16S rDNA comprising the sequence SEQ ID NO 1.

In another aspect, embodiments of this disclosure relate to an isolated *Thermoanaerobacter* sp. DIB004G characterized by having a 16S rDNA sequence at least 99 percent, preferably 99,5 to 99,99 percent identical to SEQ ID NO 1.

In still another aspect, embodiments of this disclosure relate to an isolated *Thermoanaerobacter* sp. cell comprising a 16S rDNA comprising the sequence SEQ ID NO 2.

In another aspect, embodiments of this disclosure relate to an isolated *Thermoanaerobacter* sp. DIB087G characterized by having a 16S rDNA sequence at least 99 percent, preferably 99,5 to 99,99 percent identical to SEQ ID NO 2.

In still another aspect, embodiments of this disclosure relate to an isolated *Thermoanaerobacter* sp. cell comprising a 16S rDNA comprising the sequence SEQ ID NO 3.

In another aspect, embodiments of this disclosure relate to an isolated *Thermoanaerobacter* sp. DIB097X characterized by having a 16S rDNA sequence at least 99 percent, preferably 99,5 to 99,99 percent identical to SEQ ID NO 3.

In still another aspect, embodiments of this disclosure relate to an isolated *Thermoanaerobacter* sp. cell comprising a 16S rDNA comprising the sequence SEQ ID NO 4.

In another aspect, embodiments of this disclosure relate to an isolated *Thermoanaerobacter* sp. DIB101G characterized by having a 16S rDNA sequence at least 99 percent, preferably 99,5 to 99,99 percent identical to SEQ ID NO 4.

In still another aspect, embodiments of this disclosure relate to an isolated *Thermoanaerobacter* sp. cell comprising a 16S rDNA comprising the sequence SEQ ID NO 5.

In another aspect, embodiments of this disclosure relate to an isolated *Thermoanaerobacter* sp. DIB101X characterized by having a 16S rDNA sequence at least 99 percent, preferably 99,5 to 99,99 percent identical to SEQ ID NO 5.

In still another aspect, embodiments of this disclosure relate to an isolated *Thermoanaerobacter* sp. cell comprising a 16S rDNA comprising the sequence SEQ ID NO 6.

In another aspect, embodiments of this disclosure relate to an isolated *Thermoanaerobacter* sp. DIB103X characterized by having a 16S rDNA sequence at least 99 percent, preferably 99,5 to 99,99 percent identical to SEQ ID NO 6.

In still another aspect, embodiments of this disclosure relate to an isolated *Thermoanaerobactersp.* cell comprising a 16S rDNA comprising the sequence SEQ ID NO 7.

In another aspect, embodiments of this disclosure relate to an isolated *Thermoanaerobacter* sp. DIB104X characterized by having a 16S rDNA sequence at least 99 percent, preferably 99,5 to 99,99 percent identical to SEQ ID NO 7.

In still another aspect, embodiments of this disclosure relate to an isolated *Thermoanaerobacter* sp. cell comprising a 16S rDNA comprising the sequence SEQ ID NO 8.

In another aspect, embodiments of this disclosure relate to an isolated *Thermoanaerobacter* sp. DIB107 characterized by having a 16S rDNA sequence at least 99 percent, preferably 99,5 to 99,99 percent identical to SEQ ID NO 8.

In another aspect the present invention relates to an isolated strain comprising a *Thermoanaerobacter* sp. cell according to any of the preceding aspects.

In a further aspect, embodiments of this disclosure relate to microorganism of the strain *Thermoanaerobacter* sp. DIB004G deposited as DSM 25179, a microorganism derived there from or a *Thermoanaerobacter* sp. DIB004G homolog or mutant.

In a further aspect, embodiments of this disclosure relate to microorganism of the strain *Thermoanaerobacter* sp. DIB087G deposited as DSM 25777, a microorganism derived there from or a *Thermoanaerobacter* sp. DIB087G homolog or mutant.

In a further aspect, embodiments of this disclosure relate to microorganism of the strain *Thermoanaerobacter* sp. DIB097X deposited as DSM 25308, a microorganism derived there from or a *Thermoanaerobacter* sp. DIB097X homolog or mutant.

In a further aspect, embodiments of this disclosure relate to microorganism of the strain *Thermoanaerobactersp.* DIB101G deposited as DSM 25180, a microorganism derived there from or a *Thermoanaerobacter* sp. DIB101G homolog or mutant.

In a further aspect, embodiments of this disclosure relate to microorganism of the strain *Thermoanaerobactersp.* DIB101X deposited as DSM 25181, a microorganism derived there from or a *Thermoanaerobacter* sp. DIB101X homolog or mutant.

In a further aspect, embodiments of this disclosure relate to microorganism of the strain *Thermoanaerobacter* sp. DIB103X deposited as DSM 25776, a microorganism derived there from or a *Thermoanaerobacter* sp. DIB103X homolog or mutant.

In a further aspect, embodiments of this disclosure relate to microorganism of the strain *Thermoanaerobacter* sp. DIB104X deposited as DSM 25778, a microorganism derived there from or a *Thermoanaerobacter* sp. DIB104X homolog or mutant.

In a further aspect, embodiments of this disclosure relate to microorganism of the strain *Thermoanaerobacter* sp. DIB107X deposited as DSM 25779, a microorganism derived there from or a *Thermoanaerobacter* sp. DIB107X homolog or mutant.

In another aspect the present disclosure relates to methods of producing one or more fermentation products comprising culturing one or more cells or strains according to the disclosure under suitable conditions.

In still another aspect, embodiments of this disclosure relate to methods for converting lignocellulosic hydrolysates to a biofuel or another carbon-based chemical, comprising the step of contacting the lignocellulosic hydrolysates with a microbial culture for a period of time at an initial temperature and an initial pH, thereby producing an amount of biofuel and/or other carbon-based products; wherein the microbial culture comprises an extremely thermophilic microorganism of the genus *Thermoanaerobacter,* in particular any microorganism of the strain *Thermoanaerobacter* sp. listed in table 1 with their respective accession numbers, microorganisms derived there from, mutants or homologous thereof.

In another aspect, embodiments of this disclosure relate to methods for converting starch or starch-containing feedstock to a biofuel or another carbon-based chemical, comprising the step of contacting the starch-containing feedstock with a microbial culture for a period of time at an initial temperature and an initial pH, thereby producing an amount of biofuel and/or other carbon-based products; wherein the microbial culture comprises an extremely thermophilic microorganism of the genus *Thermoanaerobacter,* in particular any microorganism of the strain *Thermoanaerobacter* sp. listed in table 1 with their respective accession numbers, microorganisms derived there from, mutants or homologous thereof.

In still another aspect, embodiments of this disclosure relate to methods for converting a combination or mixture of lignocellulosic hydrolysates and starch-containing feedstock to a biofuel or another carbon-based chemical, comprising the step of contacting the mixture with a microbial culture for a period of time at an initial temperature and an initial pH, thereby producing an amount of biofuel and/or other carbon-based products; wherein the microbial culture comprises an extremely thermophilic microorganism of the genus *Thermoanaerobacter,* in particular any microorganism of the strain *Thermoanaerobacter* sp. listed in table 1 with their respective accession numbers, microorganisms derived there from, mutants or homologous thereof.

Further, embodiments of this disclosure relate to compositions for converting lignocellulosic hydrolysates or a microbial culture comprising a cell, strain or microorganism according to the present disclosure.

Further, embodiments of this disclosure relate to the use of a cell, strain, microorganism and/or a microbial culture according to the present disclosure for the production of ethanol and/or lactic acid, a salt or an ester thereof

It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

### BRIEF DESCRIPTION OF THE DRAWINGS

FIG. 1 illustrates a phylogenetic tree based on 16S rDNA genes for all *Thermoanaerobacter sp.* strains comprised in the disclosure as listed in table 1

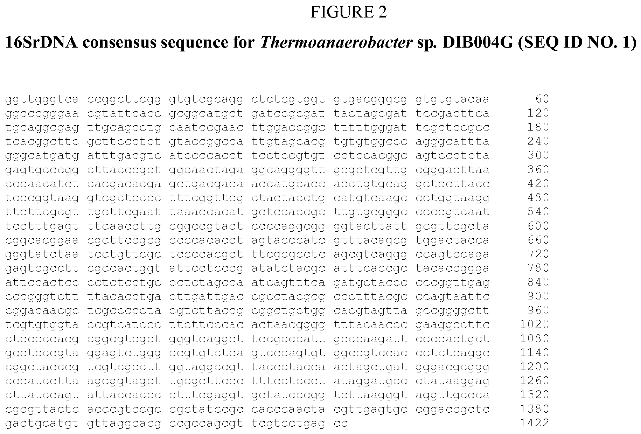

FIG. 2 shows a 16S rDNA from *Thermoanaerobacter* sp. DIB004G cell.

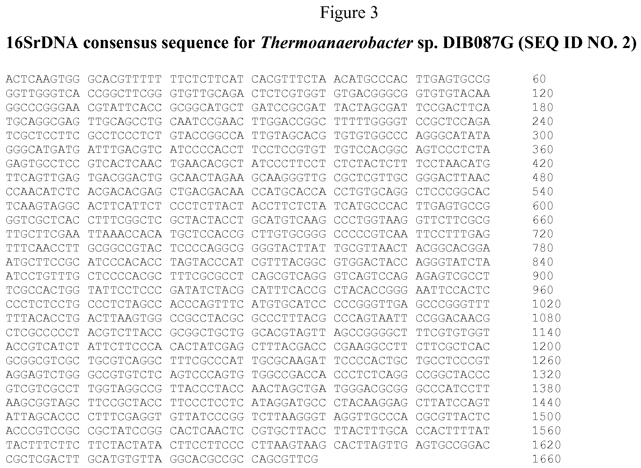

FIG. 3 shows a 16S rDNA from *Thermoanaerobacter* sp. DIB087G cell.

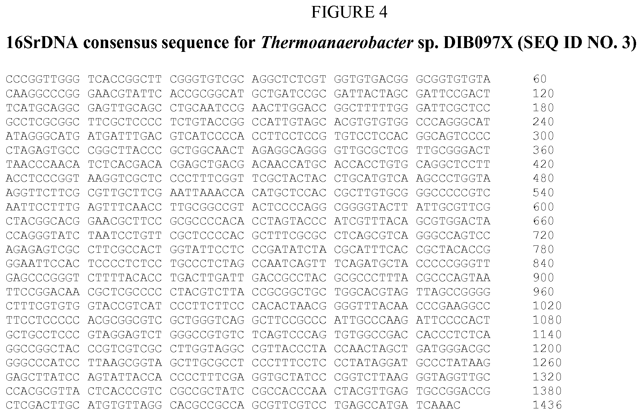

FIG. 4 shows a 16S rDNA from *Thermoanaerobacter* sp. DIB097X cell.

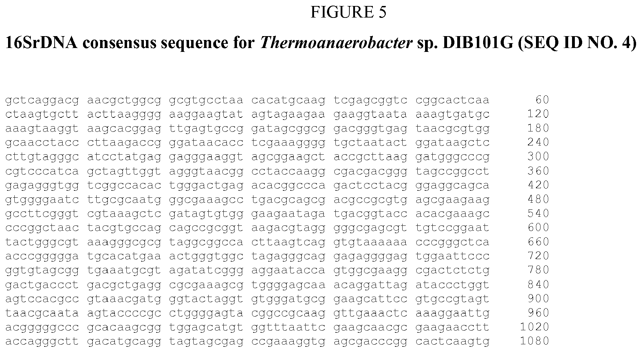

FIG. 5 shows a 16S rDNA from *Thermoanaerobacter* sp. DIB101G cell.

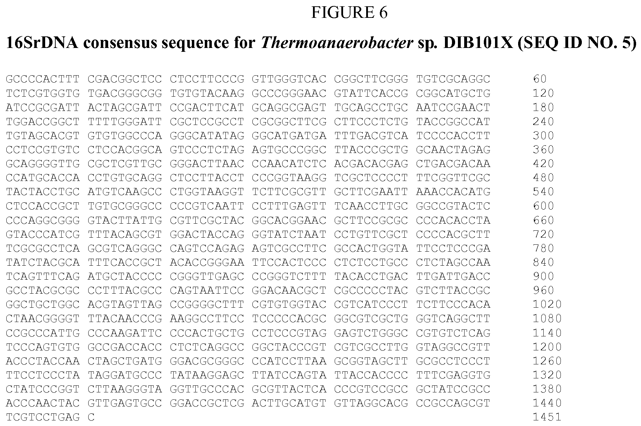

FIG. 6 shows a 16S rDNA from *Thermoanaerobacter* sp. DIB101X cell.

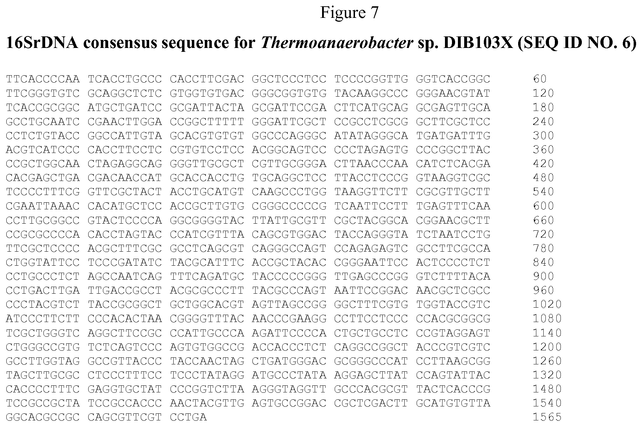

FIG. 7 shows a 16S rDNA from *Thermoanaerobacter* sp. DIB103X cell.

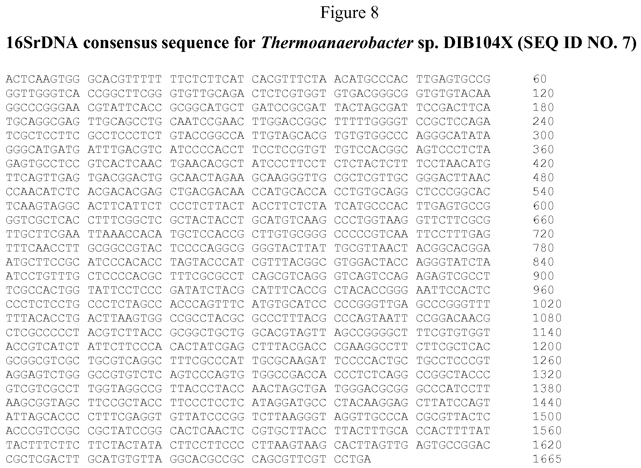

FIG. 8 shows a 16S rDNA from *Thermoanaerobacter* sp. DIB104X cell.

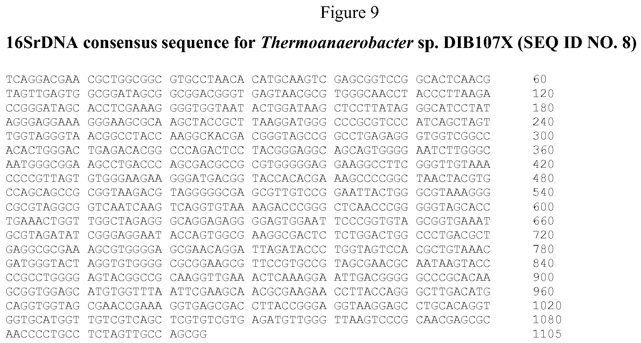

FIG. 9 shows a 16S rDNA from *Thermoanaerobacter* sp. DIB107X cell.
FIG. 10 shows a table indicating performance data from all strains listed in table 1 during cultivation on cellobiose, glucose, xylane and xylose.
FIG. 11 shows a table indicating performance data from all strains listed in table 1 during cultivation on pretreated poplar wood and performance data from selected strains DIB004G and DIB097X on different lignocellulosic feedstock types.
FIG. 12 shows a graph displaying formation of ethanol, lactate and acetate during growth of *Thermoaanerobacter* sp. DIB097X on pretreated miscanthus grass
FIG. 13 shows a graph displaying formation of ethanol, lactate and acetate during growth of Thermoanaerobacter sp. DIB004C on ground corn seed

### DETAILED DESCRIPTION OF THIS DISCLOSURE

As mentioned above, the present disclosure relates to methods, microorganisms, and compositions useful for processing lignocellulosic hydrolysates. The disclosure relates, in certain aspects, to microorganisms which are able to convert pretreated lignocellulosic biomass such as, for example, poplar wood chips or miscanthus grass, to an economically desirable product such as, for example, a biofuel (e.g., an alcohol and/or hydrogen gas (H2)), polymer, or commodity carbon-based chemical like lactic acid. Furthermore, the present disclosure relates to methods, microorganisms, and compositions useful for converting sugars like poly-, oligo, di- and/or mono-saccharides, in particular poly-, oligo, di- and/or mono- saccharides of hexoses and/or poly-, oligo, di- and/or monosaccharides of pentoses to produce carbon based chemicals like ethanol and/or lactic acid.

The present inventors have found microorganisms of the genus *Thermoanaerobacter* which have a variety of advantageous properties for their use in the conversion of oligosaccharides, disaccharides and/or monosaccharides of hexoses and polysaccharides, oligosaccharides, disaccharides and/or monosaccharides of pentoses, in particular derived from lignocellulosic hydrolysates to high level of ethanol and/or lactic acid while producing low level of acetic acid.

It is an advantage of the microorganisms according to the present disclosure that the microorganisms are able to convert highly complex polysaccharides like xylan to high yields of carbon based chemicals like ethanol and/or lactic acid.

In particular, these microorganisms are extreme thermophiles and show a broad substrate specificities and high natural production of ethanol as well as lactic acid. Moreover, ethanol and lactic acid fermentation at high temperatures, for example over 70 °C has many advantages over mesophilic fermentation. One advantage of thermophilic fermentation is the minimization of the problem of contamination in continuous cultures, since only a few microorganisms are able to grow at such high temperatures in un-detoxified lignocellulose hydrolysate.

In the present context the term "lignocellulosic hydrolysate" is intended to designate a lignocellulosic biomass which has been subjected to a pre-treatment step whereby lignocellulosic material has been at least partially separated into cellulose, hemicellulose and lignin thereby having increased the surface area of the material. The lignocellulosic material may typically be derived from plant material, such as straw, hay, garden refuse, comminuted wood, fruit hulls and seed hulls.

The term "a microorganism" as used herein may refer to only one unicellular organism as well as to numerous single unicellular organisms. For example, the term "a microorganism of the genus *Thermoanaerobacter" may* refer to one single *Thermoanaerobacter* bacterial cell of the genus *Thermoanaerobacter* as well as to multiple bacterial cells of the genus *Thermoanaerobacter*.

The terms "a strain of the genus *Thermoanaerobacter*" and "a *Thermoanaerobacter* cell" are used synonymously herein. In general, the term "microorganisms" refers to numerous cells. In particular, said term refers to at least 103 cells, preferably at least 104 cells, at least 105 or at least 106 cells.

A strain "homolog" as used herein is considered any bacterial strain, which is not significantly different by means of DNA homology as defined above and exhibits same or comparable physiological properties as described in the examples herein.

The term "mutant" as used herein refers to a bacterial cell in which the genome, including one or more chromosomes or potential extra-chromosomal DNA, has been altered at one or more positions, or in which DNA has been added or removed.

As used herein "mutant" or "homolog" means also a microorganism derived from the cells or strains according to the present disclosure, which are altered due to a mutation. A mutation is a change produced in cellular DNA, which can be spontaneous, caused by an environmental factor or errors in DNA replication, or induced by physical or chemical conditions. The processes of mutation included in this and indented subclasses are processes directed to production of essentially random changes to the DNA of the microorganism including incorporation of exogenous DNA. All mutants of the microorganisms comprise the advantages of being extereme thermophile (growing and fermenting at temperatures above 70°C) and are capable of fermenting lignocellulosic biomass to ethanol and/or lactic acid. In an advantageous embodiment, mutants of the microorganisms according to the present disclosure have in a DNA-DNA hybridization assay, a DNA-DNA relatedness of at least 80%, preferably at least 90%, at least 95%, more preferred at least 98%, most preferred at least 99%, and most preferred at least 99,9% with one of the isolated bacterial strains DIB004G, DIB087G, DIB097X, DIB101G, DIB101X, DIB103X, DIB104X and DIB107X.

The term "progeny" is refers to a product of bacterial reproduction, a new organism produced by one or more parents.

As mentioned above lignocellolytic biomass according to the present disclosure can be grass, switch grass, cord grass, rye grass, reed canary grass, mixed prairie grass, miscanthus, Napier grass, sugar-methoding residues, sugarcane bagasse, sugarcane straw, agricultural wastes, rice straw, rice hulls, barley straw, corn cobs, cereal straw, wheat straw, canola straw, oat straw, oat hulls, corn fiber, stover, soybean stover, corn stover, forestry wastes, recycled wood pulp fiber, paper sludge, sawdust, hardwood, softwood, pressmud from sugar beet, cotton stalk, banana leaves, oil palm residues and lignocellulosic biomass material obtained through processing of food plants. In advantageous embodiments, the lignocellulosic biomass material is hardwood and/or softwood, preferably poplar wood. In advantageous embodiments, the lignocellulosic biomass material is a grass or perennial grass, preferably miscanthus.

The term "DNA-DNA relatedness" in particularly refers to the percentage similarity of the genomic or entire DNA of two microorganisms as measured by the DNA-DNA hybridization / renaturation assay according to De Ley et al. (1970) Eur. J. Biochem. 12, 133-142 or Huß et al. (1983) Syst. Appl.Microbiol. 4, 184-192. In particular, the DNA-DNA hybridization assay preferably is performed by the DSMZ (Deutsche Sammlung von Mikroorganismen und Zellkulturen GmbH, Braunschweig, Germany) Identification Service.

The term "16S rDNA gene sequence similarity" in particular refers to the percentage of identical nucleotides between a region of the nucleic acid sequence of the 16S ribosomal RNA (rDNA) gene of a first microorganism and the corresponding region of the nucleic acid sequence of the 16S rDNA gene of a second microorganism. Preferably, the region comprises at least 100 consecutive nucleotides, more preferably at least 200 consecutive nucleotides, at least 300 consecutive nucleotides or at least 400 consecutive nucleotides, most preferably about 480 consecutive nucleotides.

The strains according to disclosure have the potential to be capable of producing a number of different fermentation products, including acids, alcohols, ketones and hydrogen. In one embodiment, the alcohol is selected from ethanol, butanol, propanol, methanol, propanediol and butanediol. In a further embodiment the acid is lactic acid, propionic acid, acetic acid, succinic acid, butyric acid or formic acid and the ketone is acetone.

In advantageous embodiments, the lignocellulosic biomass material is subjected to mechanical, thermochemical, and/or biochemical pretreatment. The lignocellulosic biomass material could be exposed to steam treatment. In further embodiments, the lignocellulosic biomass material is pretreated with mechanical comminution and a subsequent treatment with lactic acid, acetic acid, sulfuric acid or sulfurous acid or their respective salts or anhydrides under heat and pressure with or without a sudden release of pressure. In another embodiment, the lignocellulosic biomass material is pretreated with mechanical comminution and a subsequent treatment with either sodium hydroxide, ammonium hydroxide, calcium hydroxide or potassium hydroxide under heat and pressure with or without a sudden release of pressure.

In advantageous embodiments, the lignocellulosic biomass material is pretreated with mechanical comminution and subsequent exposure to a multi-step combined pretreatment process. Such multi-step combined pretreatment may include a treatment step consisting of cooking in water or steaming of the lignocellulosic biomass material at a temperature of 100 - 200 °C for a period of time in between 5 and 120 min. Suitable catalysts include lactic acid, acetic acid, sulfuric acid, sulfurous acid, sodium hydroxide, ammonium hydroxide, calcium hydroxide or potassium hydroxide or their respective salts or anhydrides may or may not be added to the process. The process may further include a step comprising a liquid-solid separation operation, e.g. filtration, separation, centrifugation or a combination thereof, separating the process fluid containing partially or fully hydrolyzed and solubilized constituents of the lignocellulosic biomass material from the remaining insoluble parts of the lignocellulosic biomass. The process may further include a step comprising washing of the remaining lignocellulosic biomass material. The solid material separated from solubilized biomass constituents may then be treated in a second step with steam under heat and pressure with or without a sudden release of pressure at a temperature of 150 - 250 °C for a period of time in between 1 and 15 min. In order to increase pretreatement effectiveness, a suitable catalyst includes lactic acid, acetic acid, sulfuric acid, sulfurous acid, sodium hydroxide, ammonium hydroxide, calcium hydroxide or potassium hydroxide or their respective salts or anhydrides may be added also to the second step.

In advantageous embodiments, the lignocellulosic biomass is milled before converted into biofuels like ethanol and/or carbon-based chemicals like lactic acid. In one embodiment, the lignocellulosic biomass is pretreated biomass from *Populus* sp, preferably pretreated with steam pretreatment or multi-step combined pretreatment. In another embodiment, the lignocellulosic biomass is pretreated biomass from any perennial grass, e.g. *Miscanthus* sp., preferably treated with steam pretreatment or multi-step combined pretreatment.

In further advantageous embodiments the lignocellulosic hydrolysate is then treated with an enzymatic hydrolysis with one or more appropriate carbohydrase enzymes such as cellulases, glucosidases and/or hemicellulases including xylanases.

The pretreatment method most often used is steam pretreatment, a process comprising heating of the lignocellulosic material by steam injection to a temperature of 130-230 degrees centigrade with or without subsequent sudden release of pressure. Prior to or during steam pretreatment, a catalyst like a mineral or organic acid or a caustic agent facilitating disintegration of the biomass structure can be added optionally. Catalysts often used for such a pretreatment include sulphuric acid, sulphurous acid, hydrochloric acid, acetic acid, lactic acid, sodium hydroxide (caustic soda), potassium hydroxide, calcium hydroxide (lime), ammonia or the respective salts or anhydrides of any of these agents.

Such steam pretreatment step may or may not be preceded by another treatment step including cooking of the biomass in water or steaming of the biomass at temperatures of 100 - 200 °C with or without the addition of a suitable catalyst like a mineral or organic acid or a caustic agent facilitating disintegration of the biomass structure. In between the cooking step and the subsequent steam pretreatment step one or more liquid-solid-separation and washing steps can be introduced to remove solubilized biomass components in order to reduce or prevent formation of inhibitors during the subsequent steam pretreatment step. Inhibitors formed during heat or steam pretreatment include furfural formed from monomeric pentose sugars, hydroxymethylfurfural formed from monomeric hexose sugars, acetic acid, levulinic acid, phenols and phenol derivatives.

Another type of lignocellulose hydrolysis is acid hydrolysis, where the lignocellulosic material is subjected to an acid such as sulfuric acid or sulfurous acid whereby the sugar polymers cellulose and hemicellulose are partly or completely hydrolysed to their constituent sugar monomers. A third method is wet oxidation wherein the material is treated with oxygen at 150-185 degrees centigrade. The pretreatments can be followed by enzymatic hydrolysis to complete the release of sugar monomers. This pre-treatment step results in the hydrolysis of cellulose into glucose while hemicellulose is transformed into the pentoses xylose and arabinose and the hexoses glucose, mannose and galactose. The pretreatment step may in certain embodiments be supplemented with treatment resulting in further hydrolysis of the cellulose and hemicellulose. The purpose of such an additional hydrolysis treatment is to hydrolyze oligosaccharide and possibly polysaccharide species produced during the acid hydrolysis, wet oxidation, or steam pretreatment of cellulose and/or hemicellulose origin to form fermentable sugars (e.g. glucose, xylose and possibly other monosaccharides). Such further treatments may be either chemical or enzymatic. Chemical hydrolysis is typically achieved by treatment with an acid, such as treatment with aqueous sulphuric acid or hydrochloric acid, at a temperature in the range of about 100-150 degrees centigrade. Enzymatic hydrolysis is typically performed by treatment with one or more appropriate carbohydrase enzymes such as cellulases, glucosidases and hemicellulases including xylanases.

It has been found that the microorganisms according to the present disclosure can grow efficiently on various types of pretreated and untreated biomass (e.g. wood incl. poplar, spruce and cotton wood; various types of grasses and grass residues incl. miscanthus, wheat straw, sugarcane bagasse, corn stalks, corn cobs, whole corn plants, sweet sorghum).

As used herein "efficient" growth refers to growth in which cells may be cultivated to a specified density within a specified time.

The microorganisms according to the present disclosure can grow efficiently on hydrolysis products of cellulose (e.g. disaccharide cellobiose), cellulose derived hexoses (e.g. glucose), unhydrolyzed hemicelluloses like xylan, hemicellulose derived pentoses (e.g. xylose), unhydrolyzed amyloseas well as steam pretreated poplar or miscanthus. In particular, the main products when grown on cellobiose, glucose and xylose may be ethanol and lactic acids. The main product when grown on pretreated biomass substrates was ethanol, for example, when the microorganisms were grown on steam-pretreated poplar wood or miscanthus grass the ethanol yield is high. The microorganisms according to the present disclosure also grow efficiently on cellobiose.

Cellobiose is a disaccharide derived from the condensation of two glucose molecules linked in a β(1→4) bond. It can be hydrolyzed to give glucose. Cellobiose has eight free alcohol (OH) groups, one either linkage or two hemiacetal linkages, which give rise to strong inter- and intramolecular hydrogen bonds. It is a type of dietary carbohydrate also found in mushrooms. Xylan is a generic term used to describe a wide variety of highly complex polysaccharides that are found in plant cell walls and some algae. Xylans are polysaccharides made from units of xylose.

Furthermore, the microorganisms according to the present disclosure grew efficiently on the soluble materials obtained after heat treating of lignocellulosic biomass.

It was surprisingly found that the bacterial subspecies according to the present disclosure is capable of growing in a medium comprising a lignocellulosic hydrolysates having a dry- matter content of at least 10 percent wt/wt, such as at least 15 percent wt/wt, including at least 20 percent wt/wt, and even as high as at least 25 percent wt/wt.

The microorganisms according to the disclosure are anaerobic thermophile bacteria, and they are capable of growing at high temperatures even at or above 70 degrees centigrade The fact that the strains are capable of operating at this high temperature is of high importance in the conversion of the lignocellulosic hydrolysates into fermentation products. The conversion rate of carbohydrates into e.g. ethanol and/or lactic is much faster when conducted at high temperatures. For example, the volumetric ethanol productivity of a thermophilic *Bacillus* is up to ten-fold higher than a conventional yeast fermentation process which operates at 30 degrees centigrade. Consequently, a smaller production plant is required for a given plant capacity, thereby reducing plant construction costs. As also mentioned previously, the high temperature reduces the risk of contamination from other microorganisms, resulting in less downtime, increased plant productivity and a lower energy requirement for feedstock sterilization. The high operation temperature may also facilitate the subsequent recovery of the resulting fermentation products.

Lignocellulosic biomass material and lignocellulose hydrolysates contain inhibitors such as furfural, phenols and carboxylic acids, which can potentially inhibit the fermenting organism. Therefore, it is an advantage of the microorganisms according to the present disclosure that they are tolerant to these inhibitors.

The microorganisms according to the present disclosure are novel species of the genus *Thermoanaerobacter* or novel subspecies of *Thermoanaerobacter mathranii*.

For example, the genus *Thermoanaerobacter* includes different species of extremely thermophilic (temperature optima for growth higher than 70 °C) hemicellulolytic and saccharolytic strictly anaerobic bacteria (Lee et al. 1993). *Thermoanaerobactermathranii* DSM 11426 is an extremely thermophilic bacterium. It has a temperature optimum between 70 and 75 °C and was isolated from a hot spring in Iceland (Larsen et al. 1997). It uses a number of sugars including xylan as carbon sources, but did not utilize microcrystalline cellulose. Fermentation end products on xylose were ethanol, acetate, low amounts of lactate, CO₂, and H₂ (Larsen et al. 1997).

According to the present disclosure, the microorganisms produce ethanol and lactic acid and show several features that distinguish them from currently used microorganisms: (i) high yield and low product inhibition, (ii) simultaneous utilization of lignocellolosic biomass material derived sugars, and (iii) growth at elevated temperatures. The microorganisms according to the present disclosure are robust thermophilic organisms with a decreased risk of contamination. They efficiently convert an extraordinarily wide range of biomass components to carbon-based chemicals like ethanol or lactic acid.

As mentioned above, in one aspect, the present disclosure relates to an isolated cell comprising a 16S rDNA sequence selected from the group consisting of: SEQ ID NO 1, SEQ ID NO 2, SEQ ID NO 3, SEQ ID NO 4, SEQ ID NO 5, SEQ ID NO 6, SEQ ID NO 7, SEQ ID NO 8 and a combination of any thereof.

In one aspect, the present disclosure pertains to an isolated *Thermoanaerobacter* sp. cell having a 16S rDNA sequence at least 99, at least 99,3, at least 99,5, at least, 99,7, at least 99,9, at least 99,99 percent identical to SEQ ID NO 1, SEQ ID NO 2, SEQ ID NO 3, SEQ ID NO 4, SEQ ID NO 5, SEQ ID NO 6, SEQ ID NO 7 and/or SEQ ID NO 8.

In one embodiment of the present disclosure the isolated cell is *Thermoanaerobacter* sp. DIB004G (DSMZ Accession number 25179), cells derived there from, mutants there from, a progeny or a *Thermoanaerobacter* sp. DIB004G homolog or mutant.

In another embodiment of the present disclosure the isolated cell is *Thermoanaerobacter* sp. DIB087G (DSMZ Accession number 25777), cells derived there from, mutants there from, a progeny or a *Thermoanaerobacter* sp. DIB087G homolog or mutant.

In another embodiment of the present disclosure the isolated cell is *Thermoanaerobacter* sp. DIB097X (DSMZ Accession number 25308), cells derived there from, mutants there from, a progeny or a *Thermoanaerobacter* sp. DIB097X homolog or mutant.

In another embodiment of the present disclosure the isolated cell is *Thermoanaerobacter* sp. DIB101G (DSMZ Accession number 25180), cells derived there from, mutants there from, a progeny or a *Thermoanaerobacter* sp. DIB101G homolog or mutant.

In another embodiment of the present disclosure the isolated cell is *Thermoanaerobacter* sp. DIB101X (DSMZ Accession number 25181), cells derived there from, mutants there from, a progeny or a *Thermoanaerobacter* sp. DIB101X homolog or mutant.

In another embodiment the isolated cell is *Thermoanaerobacter* sp. DIB103X (DSMZ Accession number 25776), cells derived there from, mutants there from, a progeny or a *Thermoanaerobacter* sp. DIB103X homolog or mutant.

In another embodiment the isolated cell is *Thermoanaerobacter* sp. DIB104X (DSMZ Accession number 25778), cells derived there from, mutants there from, a progeny or a *Thermoanaerobacter* sp. DIB104X homolog or mutant.

In another embodiment the isolated cell is *Thermoanaerobacter* sp. DIB107X (DSMZ Accession number 25779), cells derived there from, mutants there from, a progeny or a *Thermoanaerobacter* sp. DIB107X homolog or mutant.

The disclosure is based on the isolated bacterial strains *Thermoanaerobacter* sp. as listed in table 1 that contain 16S rDNA sequences 100 percent and/or 99.99 percent identical to the respectively list sequences.

**Table 1**

| Genus | Species | Name | DSMZ accession number | Deposition date | 16SrDNA SEQ ID NO. |
|---|---|---|---|---|---|
| Thermoanaerobacter | sp. | DIB004G | DSM 25179 | 15.09.2011 | 1 |
| Thermoanaerobacter | sp. | DIB087G | DSM 25777 | 15.03.2012 | 2 |
| Thermoanaerobacter | sp. | DIB097X | DSM 25308 | 27.10.2011 | 3 |
| Thermoanaerobacter | sp. | DIB101G | DIB 25180 | 15.09.2011 | 4 |
| Thermoanaerobacter | sp. | DIB101X | DSM 25181 | 15.09.2011 | 5 |
| Thermoanaerobacter | sp. | DIB103X | DSM 25776 | 15.03.2012 | 6 |
| Thermoanaerobacter | sp. | DIB104X | DSM 25778 | 15.03.2012 | 7 |
| Thermoanaerobacter | sp. | DIB107X | DSM 25779 | 15.03.2012 | 8 |

All strains as listed in table 1 have been deposited in accordance with the terms of the Budapest Treaty on September 15, 2011 with DSMZ - Deutsche Sammlung von Mikroorganismen und Zellkulturen GmbH, Inhoffenstr. 7B, 38124 Braunschweig, Germany - under the respectively indicated accession numbers and deposition dates by DIREVO Industrial Biotechnology GmbH, Nattermannallee 1, 50829 Cologne, Germany (DE).

As is apparent from the following, the preferred strains of the present disclosure have been deposited. Other cells, strains, bacteria, microorganisms and/or microbial cultures of the present disclosure can therefore be obtained by mutating the deposited strains and selecting derived mutants having enhanced characteristics. Desirable characteristics include an increased range of sugars that can be utilized, increased growth rate, ability to produce higher amounts of fermentation products such as ethanol and/or lactic acid, etc. Suitable methods for mutating bacteria strains and selecting desired mutants are described in Functional analysis of Bacterial genes: A practical Manual, edited by W. Schumann, S. D. Ehrlich & N. Ogasawara, 2001.

The microorganisms of the species *Thermoanaerobacter* sp. according to the present disclosure in particular refer to a microorganism which belongs to the genus *Thermoanaerobacter* and which preferably has one or more of the following characteristics:
a) it is a microorganism of the genus *Thermoanaerobacter,* and/or
b) in a DNA-DNA hybridization assay, it shows a DNA-DNA relatedness of at least 70%, preferably at least 90%, at least 95%, more preferred at least 98%, most preferred at least 99% with the *Thermoanaerobacter* sp. strains listed in table 1 with their respective accession numbers; and/or
c) it displays a level of 16S rDNA gene sequence similarity of at least 98%, preferably at least 99% or at least 99,5%, more preferably 100% with either of the *Thermoanaerobacter* sp. strains listed in table 1 with their respective accession numbers; and/or
d) it is capable of surviving and/or growing and/or producing high levels of at least one fermentation product at high temperature conditions above 70 °C, and/or
e) it is a Gram-positive bacterium; and/or
f) it is saccharolytic thermophilic microorganism; and/or
g) it is a xylanolytic thermophilic microorganism.

Preferably, at least two or at least three, and more preferred all of the above defined criteria a) to g) are fulfilled.

In an advantageous embodiment, the microorganisms according to the present disclosure in particular refer to a microorganism which belongs to the genus *Thermoanaerobacter* and which preferably has one or more of the following characteristics:
a) It is a microorganism of the genus *Thermoanaerobacter*
b) it is a microorganism of the species *Thermoanaerobacter thermohydrosulfuricus, Thermoanaerobacter thermocopriae* or *Thermoanaerobacter mathranii*;
c) in a DNA-DNA hybridization assay, it shows a DNA-DNA relatedness of at least 80%, preferably at least 90%, at least 95%, more preferred at least 98%, most preferred at least 99%, and most preferred at least 99,9% with one of the strains of table 1; and/or
d) it displays a level of 16S rDNA gene sequence similarity of at least 98%, preferably at least 99%, at least 99,5% or at least 99,7%, more preferably 99,99% with one of the strains listed in table 1; and/or
e) it is capable of surviving and/or growing and/or producing a fermentation product selected from the group consisting of carboxylic acids, preferably lactic acid and alcohols, preferably ethanol at temperature conditions above 70 °C, in particular of above 72 °C.

Preferably, at least two or at least three, and more preferred all of the above defined criteria a) to e) are fulfilled.

The *Thermoanaerobacter* sp. strains according to the present disclosure have several highly advantageous characteristics needed for the conversion of lignocellulosic biomass material. Thus, these base strains possess all the genetic machinery for the conversion of both pentose and hexose sugars to various fermentation products such as ethanol and lactic acid. As will be apparent from the below examples, the examination of the complete 16S rDNA sequence showed that the related strains *Thermoanaerobacter* sp. DIB087G, *Thermoanaerobacter* sp. DIB101G and *Thermoanaerobacter* sp. DIB104X may be related to *Thermoanaerobacter thermohydrosulfuricus,* although the 16S rDNA sequences clearly place them in separate subspecies or even different species. The strain *Thermoanaerobacter* sp. DIB107X may be related to *Thermoanaerobacter thermocopriae,* although the 16S rDNA sequences clearly place them in separate subspecies or even different species. The strains *Thermoanaerobacter* sp. DIB004G, *Thermoanaerobacter* sp. DIB097X, *Thermoanaerobacter* sp. DIB101X and *Thermoanaerobactersp.* DIB103X may be related to *Thermoanaerobactermathranii,* although the 16S rDNA sequences clearly place them in separate subspecies or even different species.

It is a great advantage of the *Thermoanaerobacter* sp. strains according to the present disclosure that they are xylanolytic and saccharolytic (ferment hemicelluloses, e.g. xylan, hexoses and pentoses to ethanol, lactate and small amounts of acetate).

In a preferred embodiment, the *Thermoanaerobacter* sp. microorganism is
a) Either *Thermoanaerobacter* sp. listed in table 1, deposited under their respectively indicated accession number and deposition date, according to the requirements of the Budapest Treaty at the Deutsche Sammlung von Mikroorganismen und Zellkulturen (DSMZ), Inhoffenstraße 7B, 38124 Braunschweig (DE) by DIREVO Industrial Biotechnology GmbH, Nattermannallee 1, 50829 Cologne, Germany (DE), or
b) a microorganism derived from either of these *Thermoanaerobacter* sp. strains, or
c) a homolog or mutant of either respective *Thermoanaerobacter* sp. strain

All strains *Thermoanaerobacter* sp. as listed in table 1 belong to the genus *Thermoanaerobacter* and are extremely thermophilic (growth at temperatures higher than 70 °C), xylanolytic, amylolytic and saccharolytic, strictly anaerobic, Gram-positive bacteria. Cells are straight rods 0.3-0.4 µm by 2.0-6.0 µm, occuring both singly and in pairs. These strains grow on various sugars as substrate, including starch, xylan, xylose, cellobiose, and glucose.

The main fermentation products on these substrates are ethanol and lactate. Low amounts of acetate are also formed.

In advantageous embodiments the cells, strains, microorganisms may be modified in order to obtain mutants or derivatives with improved characteristics. Thus, in one embodiment there is provided a bacterial strain according to the disclosure, wherein one or more genes have been inserted, deleted or substantially inactivated. The variant or mutant is typically capable of growing in a medium comprising a lignocellulosic biomass material and/or a lignocellulosic hydrolysate.

In another embodiment, there is provided a process for preparing variants or mutants of the microorganisms according to the present disclosure, wherein one or more genes are inserted, deleted or substantially inactivated as described herein.

In some embodiments one or more additional genes are inserting into the strains according to the present disclosure. Thus, in order to improve the yield of the specific fermentation product, it may be beneficial to insert one or more genes encoding a polysaccharase into the strain according to the invention. Hence, in specific embodiments there is provided a strain and a process according to the disclosure wherein one or more genes encoding a polysaccharase which is selected from cellulases (such as EC 3.2.1.4); beta-glucanases, including glucan-1,3 beta-glucosidases (exo-1,3 beta-glucanases, such as EC 3.2.1.58), 1,4-beta-cellobiohydrolases (such as EC 3.2.1.91) and endo-I,3(4)-beta-glucanases (such as EC 3.2.1.6); xylanases, including endo-I,4-beta-xylanases (such as EC 3.2.1.8) and xylan 1,4-beta-xylosidases (such as EC 3.2.1.37); pectinases (such as EC 3.2.1.15); alpha-glucuronidases, alpha-L-arabinofuranosidases (such as EC 3.2.1.55), acetylesterases (such as EC 3.1.1.-), acetylxylanesterases (such as EC 3.1.1.72), alpha-amylases (such as EC 3.2.1.1), beta-amylases (such as EC 3.2.1.2), glucoamylases (such as EC 3.2.1.3), pullulanases (such as EC 3.2.1.41), beta-glucanases (such as EC 3.2.1.73), hemicellulases, arabinosidases, mannanases including mannan endo-I,4-beta-mannosidases (such as EC 3.2.1.78) and mannan endo-I,6-alpha-mannosidases (such as EC 3.2.1.101), pectin hydrolases, polygalacturonases (such as EC 3.2.1.15), exopolygalacturonases (such as EC 3.2.1.67) and pectate lyases (such as EC 4.2.2.10), are inserted.

In accordance with the present disclosure, a method of producing a fermentation product comprising culturing a strain according to the invention under suitable conditions is also provided.

The strains according to the disclosure are strictly anaerobic microorganisms, and hence it is preferred that the fermentation product is produced by a fermentation process performed under strictly anaerobic conditions. Additionally, the strain according to invention is an extremely thermophillic microorganism, and therefore the process may perform optimally, when it is operated at temperature in the range of about 40-95 degrees centigrade, such as the range of about 50-90 degrees centigrade, including the range of about 60-85 degrees centigrade, such as the range of about 65-75 degrees centigrade

For the production of certain fermentation products, it may be useful to select a specific fermentation process, such as batch fermentation process, including a fed-batch process or a continuous fermentation process. Also, it may be useful to select a fermentation reactor such as an immobilized cell reactor, a fluidized bed reactor or a membrane bioreactor.

In accordance with the disclosure, the method is useful for the production of a wide range of fermentation products including acids, alcohols, ketones and hydrogen. Thus fermentation products such as ethanol, butanol, propanol, methanol, propanediol, butanediol, lactic acid, propionic acid, acetic acid, succinic acid, butyric acid, formic acid and acetone may be produced in accordance with the disclosure.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

The following methods and examples are offered for illustrative purposes only, and are not intended to limit the scope of the present disclosure in any way.

### Methods and Examples

In the following examples, materials and methods of the present disclosure are provided including the determination of properties of the strains according to the present disclosure. It should be understood that these examples are for illustrative purpose only and are not to be construed as limiting this disclosure in any manner.

### Example 1: Isolation and Cultivation

All procedures for enrichment and isolation of strains listed in table 1 employed anaerobic technique for strictly anaerobic bacteria (Hungate 1969). The strains were enriched from environmental samples at temperatures higher than 70 °C with crystalline cellulose and beech wood as substrate. Isolation was performed by serial dilutions in liquid media with xylan as substrate followed by picking colonies grown on solid agar medium at 72 °C in Hungate roll tubes (Hungate 1969).

The cells are cultured under strictly anaerobic conditions applying the following medium:

| **Basic medium** | | |
|---|---|---|
| NH4Cl | 1.0 | g |
| NaCl | 0.5 | g |
| MgSO4 x 7 H2O | 0.3 | g |
| CaCl2 x 2 H2O | 0.05 | g |
| NaHCO3 | 0.5 | g |
| K2HPO4 | 1.5 | g |
| KH2PO4 | 3.0 | g |
| Yeast extract (bacto, BD) | 0.5 | g |
| Cellobiose | 5.0 | g |
| Vitamins (see below) | 1.0 | ml |
| Trace elements (see below) | 0.5 | ml |
| Resazurin | 1,0 | mg |
| Na2S x 9 H2O | 0,75 | g |
| Distilled water | 1000.0 | ml |

| **Trace elements stock solution** | | |
|---|---|---|
| NiCl₂x6H₂O | 2 | g |
| FeSO₄x7H₂O | 1 | g |
| NH₄Fe(III) citrate, brown, 21,5% Fe | 10 | g |
| MnSO₄xH₂O | 5 | g |
| COCl₂x6H₂O | 1 | g |
| ZnSO₄x7H₂O | 1 | g |
| CuSO₄x5H₂O | 0,1 | g |
| H₃BO₃ | 0,1 | g |
| Na₂MoO₄x2H₂O | 0,1 | g |
| Na₂SeO₃x5H₂O | 0,2 | g |
| Na₂WoO₄x2H₂O | 0,1 | g |
| Distilled water | 1000.0 | ml |
| Add 0,5 ml of the trace elements stock solution to 1 liter of the medium | | |

| **Vitamine stock solution** | | |
|---|---|---|
| nicotinic acid | 200 | mg |
| cyanocobalamin | 25 | mg |
| p-aminobenzoic acid (4-aminobenzoic acid) | 25 | mg |
| calcium D-pantothenate | 25 | mg |
| thiamine-HCl | 25 | mg |
| riboflavin | 25 | mg |
| lipoic acid | 25 | mg |
| folic acid | 10 | mg |
| biotin | 10 | mg |
| pyridoxin-HCl | 10 | mg |
| Distilled water | 200.0 | ml |
| Add 1 ml of the vitamine stock solution to 1 liter of the medium | | |

All ingredients except sulfide are dissolved in deionized water and the medium is flushed with nitrogen gas (purity 99,999%) for 20 min at room temperature. After addition of sulfide, the pH-value is adjusted to 7.0 at room temperature with 1 M HCl. The medium is then dispensed into Hungate tubes or serum flasks under nitrogen atmosphere and the vessels are tightly sealed. After autoclaving at 121 °C for 20 min pH-value should be in between 6.8 and 7.0.

Soluble sugar substrates (xylose, cellobiose, glucose) as specified for individual experiments are added sterile filtered after autoclaving. Xylan is autoclaved with the medium. Subsequent to autoclaving, cultures are inoculated by injection of a seed culture through the seal septum and inoculated in an incubator at 72 °C for the time indicated.

### Example 2: HPLC

Sugars and fermentation products were quantified by HPLC-RI using a Via Hitachi LaChrom Elite (Hitachi corp.) fitted with a Rezex ROA Organic Acid H+ (Phenomenex). The analytes were separated isocratically with 2.5 mM H₂S0₄ and at 65 °C.

### Example 3: Phylogenetic analysis of 16S rDNA genes

Genomic DNA was isolated from cultures grown as described above and 16SrDNA amplified by PCR using 27F (AGAGTTTGATCMTGGCTCAG) as forward and 1492R (GGTTACCTTGTTACGACTT) as reverse primer. The resulting products were sequenced and the sequences analyzed using the Sequencher 4.10.1 software (Gene Codes Corporation). The NCBI database was used for BLAST procedures.

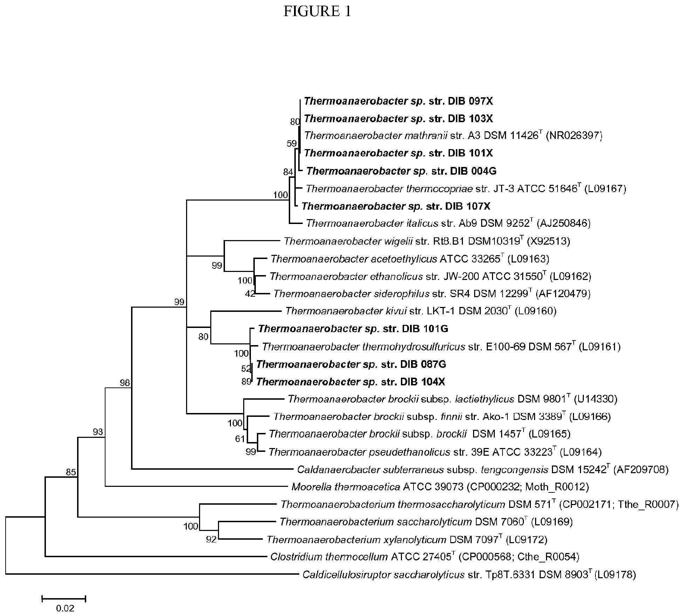

Alignment was carried out using ClustalW (Chenna et al. 2003) and the phylogenetic tree was constructed using software MEGA4 (Kumar et al. 2001). The tree for all strains listed in table 1 is displayed in figure 1.

### Example 4: Production of ethanol and lactate on different substrates

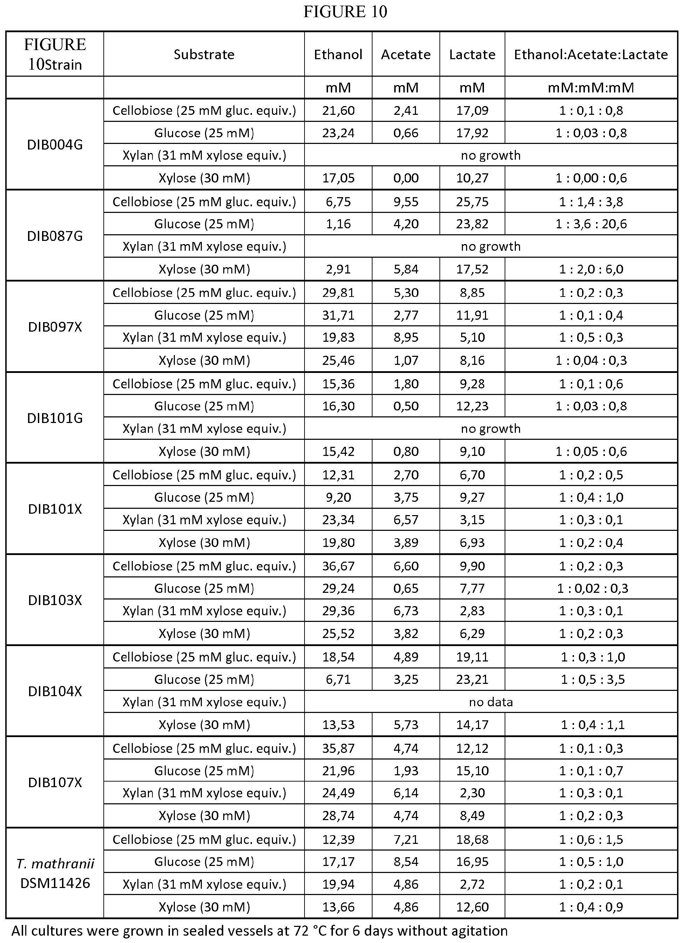

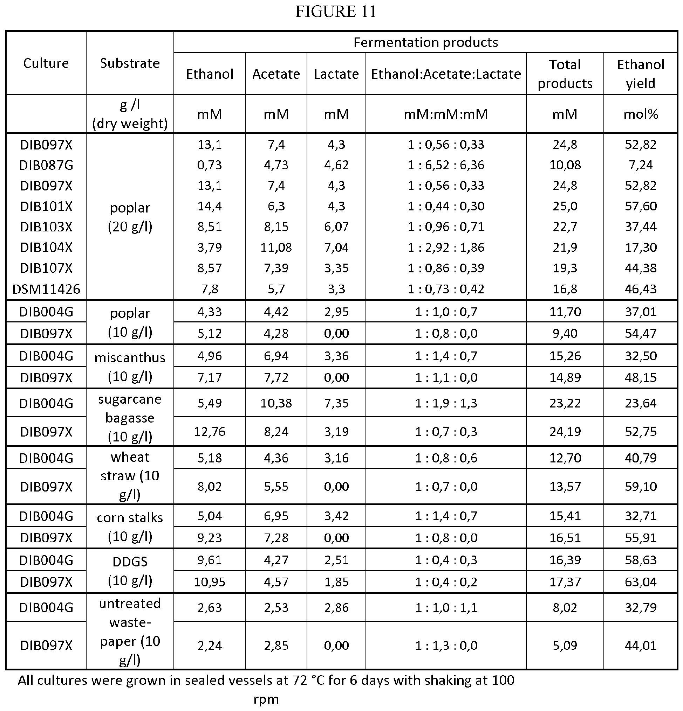

Experiments on growth and fermentation on cellobiose, glucose, xylan and xylose as well as on pretreated poplar wood, miscanthus grass, sugarcane bagasse, wheat straw, corn stalks and DDGS as well as on non-pretreated waste paper were performed by cultivation in sealed 16 ml tubes with 8 ml medium described in Example 1. All strains grew well on these substrates (Figures 10 and 11) except strains DIB004G, DIB087G and DIB101G which did not grow on xylane. No growth was detected on cellulose. The main fermentation product was ethanol followed by lactate. Only small amounts of acetate were formed (Figures 10 and 11). In contrast, the known ethanol-producing thermophilic bacterium *Thermoanaerobacter mathranii* strain A3 (DSM 11426) (Larsen et al 1997) produced lower amounts of ethanol as well as higher amounts of lactate and acetate.

### Example 5: Fermentation

Batch experiments with all strains, e.g. DIB004G, were performed by cultivation on the medium described above with addition of the respectively indicated substrate, e.g. 20 g/L miscanthus grass pretreated with a suitable method selected from those described above comprising heating in the presence of dilute acid followed by sudden release of pressure.

Temperature is controlled to 72 °C and the pH-value is controlled to 6.75 ± 0.1 throughout the fermentation. The fermenter is purged with nitrogen to remove excess oxygen before sodium sulphide is added as described above.

The fermentation is started by addition of a seed culture prepared as described in example 1.

The results of the HPLC analysis as described in example 2 show parallel production of ethanol, lactic acid and acetic acid.

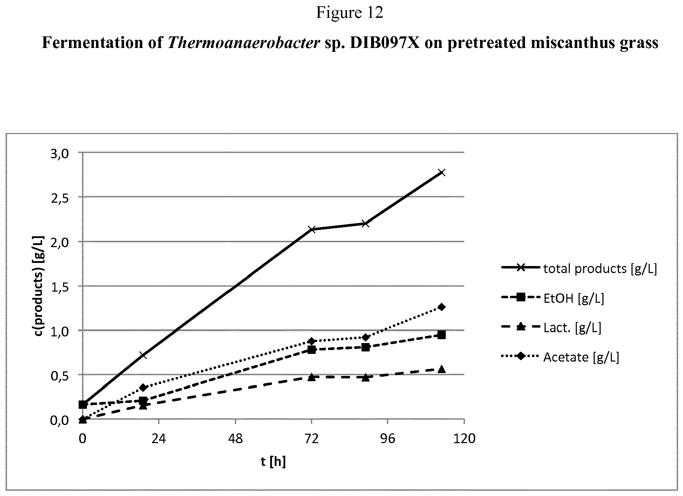

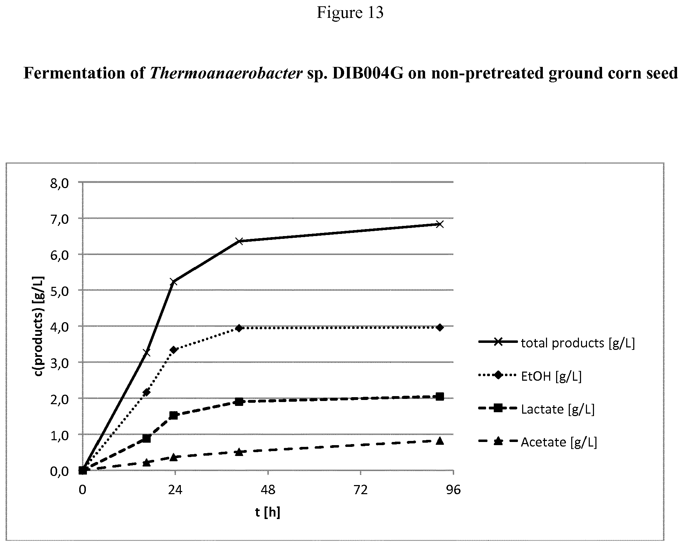

The results for product formation during a fermentation of *Thermoanaerobacter* sp. DIB097X on pretreated miscanthus grass is shown in Figure 12. The results for product formation during a fermentation of *Thermoanaerobacter* sp. DIB004G on non-pretreated ground corn seed is shown in Figure 13.

### LIST OF ADDITIONAL REFRENCES:

Lee Y-E, Jain MK, Lee c. Lowe SE, Zeikus JG (1993) Taxonomic distinction of saccharolytic thermophilic anaerobes: Description of Thermoanaerobacterium xylanolyticum gen. nov., sp. nov., and Thermoanaerobacterium saccharolyticum gen. nov., sp.nov.; Reclassification of Thermoanaerobium brockii, Clostridium thermosulfurogenes, and Clostridium thermohydrosulfiricum EIO0-69 as Thermoanaerobacter brockii comb. nov., Thermoanaerobacterium thermosulfurigenes comb. nov., and Thermoanaerobacter thermohydrosulfuricus comb. nov., respectively; and transfer of Clostridium hermohydrosulfuricum 39E to Thermoanaerobacterethanolicus. Int J Syst Bacteriol 43:41-51. Larsen L, Nielsen P, Ahring BK. (1997) Thermoanaerobacter mathranii sp. nov., an ethanol-producing, extremly thermophilic anaerobic bacterium from a hot spring in Iceland. Arch Microbiol 168:114-119.
Hungate RE. (1969) A roll tube method for cultivation of strict anaerobes. In: Methods in Microbiology Eds. Norris JR and Ribbons DW. pp 118-132. New York: Academic Press.
Chenna R, Sugawara H, Koike T, Lopez R, Gibson TJ, Higgins DG, Thompson JD. (2003) Multiple sequence alignment with the Clustal series of programs. Nucleic Acids Res. 13:3497-3500.
Kumar S, Tamura K, Jakobsen IB, Nei M. (2001) MEGA2: molecular evolutionary genetics analysis software. Bioinformatics. 17:1244-1245.
US patent US 6,555,350
International patent application WO 2007/134607
US patent US 6,555,350
International patent application WO 2007/134607

## Claims

1. An isolated *Thermoanaerobacter* sp. cell capable of growing and producing a carbon-based chemical at a temperature of above 70°C comprising a 16S rDNA sequence of SEQ ID NO 1, and wherein the cell is DIB004G deposited under the DSMZ Accession number 25179.

2. The isolated cell according to claim 1, wherein the cell is capable of producing a carbon-based chemical selected from the group consisting of carboxylic acids like lactic acid, propionic acid, acetic acid, succinic acid, malic acid, butyric acid and formic acid, or salts or esters thereof and alcohols like ethanol, butanol, propanol, methanol, propanediol and butanediol.

3. The isolated cell according to any one of the preceding claims, which is capable of growing at a temperature of above 70°C, in particular between 70 and 90°C, in particular between 70 and 85°C, in particular at a temperature between 70°C and 75°C.

## Patentansprüche

1. Eine *Thermoanaerobacter* sp. Zelle, umfassend eine 16S rDNA Sequenz mit der SEQ ID NR. 1, welche die Fähigkeit besitzt, bei einer Temperatur über 70°C zu wachsen und eine auf Kohlenstoff basierende Chemikalie zu produzieren, wobei die Zelle die DIB004G, hinterlegt unter der DSMZ Hinterlegungsnummer 25179, ist.

2. Die isolierte Zelle gemäß Anspruch 1, wobei die Zelle die Fähigkeit besitzt, eine auf Kohlenstoff basierende Chemikalie, ausgewählt aus der Gruppe bestehend aus Carbonsäuren wie Milchsäure, Propionsäure, Essigsäure, Bernsteinsäure, Äpfelsäure, Buttersäure und Ameisensäure, oder Salze oder Ester davon, und Alkohole wie Ethanol, Buthanol, Propanol, Methanol, Propandiol und Butandiol, zu produzieren.

3. Die isolierte Zelle gemäß einem der vorhergehenden Ansprüche, die fähig ist bei einer Temperatur über 70°C, insbesondere zwischen 70 und 90°C, insbesondere zwischen 70 und 85°C, insbesondere zwischen 70 und 75°C, zu wachsen.

## Revendications

1. Une cellule Thermoanaerobacter sp, comprenant une séquence d'ADNr 16S avec SEQ ID No. 1, qui a la capacité de croître à une température supérieure à 70 °C et de produire un produit chimique à base de carbone, dans lequel la cellule est la DIB004G, déposée sous le numéro de dépôt DSMZ 25179.

2. La cellule isolée selon la revendication 1, dans lequel ladite cellule a la capacité de produire un produit chimique à base de carbone choisi parmi les acides carboxyliques tels que l'acide lactique, l'acide propionique, l'acide acétique, l'acide succinique, d'acide malique, d'acide butyrique et l'acide formique ou les sels ou esters de celui-ci, et les alcools comme l'éthanol, le butanol, le propanol, le méthanol, le propanediol et butandiol.

3. La cellule isolée selon l'une quelconque des revendications précédentes, qui a la capacité de croître à une température supérieure à 70 °C, en particulier entre 70 °C et 90 °C, en particulier entre 70°C et 85°C, en particulier entre 70 °C et 75 °C.
